# EUROPEAN PATENT APPLICATION

(11) **EP 1 302 908 A2**
(43) Date of publication of application: **16.04.2003**
(21) Application number: 02252499.5
(22) Date of filing: 06.04.2002
(51) Int. Cl.: G07C 9/00

(54) **Verification techniques for biometric identification systems**

(30) Priority: 09.10.2001 JP 2001311738
(71) Applicant: BMF Corporation, Kawasaki-shi, Kanagawa 215-0034 (JP)
(72) Inventor: Tamori, Teruhiko, Iruma-shi, Saitama 358-0026 (JP)
(74) Representative: Collingwood, Anthony Robert

(57) **Abstract**

Methods and apparatus are described for determining whether an object comprises living tissue. An antenna is provided which is operable to form an electrical circuit with the object. Detection circuitry is provided which is operable to detect at least one parameter corresponding to the electrical circuit. Determination circuitry is provided which is operable to determine whether the object comprises living tissue with reference to the at least one parameter.

## Description

### BACKGROUND OF THE INVENTION

The present invention relates to technology for preventing illegal access in the field of individual authentication for determining an actual person using biometric technology such as fingerprint authentication, for example, and particularly to a methods and devices for determining authenticity of specific parts of the human body.

Traditionally, a portrait style photograph of an actual person that can be seen on a driver's license, passport or application card (slip) for a university entrance examination or the like has been widely used throughout the world as individual authentication data. A person's face is turned into image data (for example, a photograph) as individual authentication data, and it is common practice for a third party (another person) to make a determination as to authenticity by making a visual comparison of the photograph and the actual person's face.

However, with the advancement of the latest high precision duplication technology, individual authentication using this type of image will soon become obsolete. This is mainly due to the fact that it is extremely easy to make a duplicate that is almost identical to an original by comlecting a high precision scanner of about 1200 dpi and a 600 dpi color printer to a Windows (Registered Trademark) system. On the other hand, the fact that human decision is required in this type of individual authentication is one reason why it seems out of step with the multi-media and IT age. If possible, it is desirable to be able to cany out individual authentication automatically without having to rely on human judgment, e.g., using a computer or other type of automated system.

Instead of the individual authentication of the related art using portrait style photographs, there has recently been a lot of interest in individual authentication using biometrics technology. Specifically, individual authentication using biometrics such as a person's DNA, fingerprints, voice, wrist vascularity pattern, retina, iris etc. has been drawing attention, and has been partially implemented. Authentication using these types of technologies involves specifying an individual using specialized authentication technologies for parts of the human body and requires advanced authorization technology. The advantage is that unmanned decision making can be carried out using a computer without the need for a person to make the decision using a photograph. It is therefore possible to have individual specification that is simple and cheap without any difficult operations, and for this reason individual authentication technology will inevitably become widespread.

However, recently in the field of individual authentication making use of biometrics and particularly fingerprint, a fingerprint forgery problem has arisen. Specifically, the fact that falsified fingerprints have become feasible as a result of strenuous endeavors with respect to forgery technology is disclosed in the following publications (1), (2) and (3).
(1) "Will Fingerprint Matching Devices Accept Artificial Fingers?" Technical Reports Of The Electronic Information Communication Society, Vol. 100 No. 213, ISEC2000-45, Electronic Information Communication Society, July 2000
(2) "Will Fingerprint Matching Devices Accept Artificial Fingers? (Part 2)" Computer Security Symposium, 2000 collection, Data Processing Society Symposium Series, Vol. 2000 No. 12, Data Processing Society, October 2000
(3) "Will Fingerprint Matching Devices Accept Artificial Fingers?( Part 3)" Proceedings Of The 2001 Encryption And Information Security Symposium (SCIS2001), Vol. II pp719-724, Electronic Information Communication Society, January 2001

These publications disclose artificial manufacture of a finger having a fingerprint of a rubbery material (gelation of a gelatin solution), and the performing of experiments with such an artificial finger operating a fingerprint matching system containing 9 models of commercially available fingerprint matching devices. In almost all cases it was possible to operate the systems in the same way as with an actual human finger.

While it has been known to manufacture false fingerprints from silicon resin up to now, the ideas disclosed in the above publications are characterized in that a material having a moisture content that is close to that of a human finger was successfully searched, and nobody other than an engineer who knows the fact that a fingerprint sensor uses finger sweat (sweat glands) in detection of human fingerprint patterns would have been really capable of making such an invention.

This fact verifies that it is easy to manufacture a mold for a human finger (a mold that can be used time and time again) and it is therefore possible to artificially manufacture false fingers, and false fingerprints extremely cheaply.

As a result, with fingerprint matching systems using a conventional fingerprint sensor, it has been experimentally proven that it is not possible to accurately discern whether a human finger is being pressed down or an artificial finger is being pressed down, and a very serious problem has recently arisen whereby acts of illegal access or acts of impersonation are possible.

Means can be conceived of for preventing acts of illegal access or acts of impersonation using this type of fingerprint falsification, and there is a method for determining finger authenticity using medical electronics technology for measuring or detecting the pulse, blood pressure, blood oxygen level etc. of a human finger. A system for realizing such methods is, however, large in scale, and there are problems from the point of view of cost due to the fact that various application systems of several thousand yen or several tens of thousand yen are supported, and implementation on the open market for general products is difficult. The same thing can also be said for individual authentication technology using other parts of the body such as the palm of a hand or a wrist, and does not only apply to fingers.

### SUMMARY OF THE INVENTION

According to the present invention various techniques are provided which are capable of determining authenticity of parts of the human body, simply and at low cost.

According to one embodiment, the present invention provides a living body determination device having an antenna arranged in association with a specified portion of a sensor onto which a specified part of a human body is acted for obtaining data identifying an individual, and detection means for detecting characteristics of electrical output of an electrical circuit including said antenna when an object is acted on said specified portion of the sensor, wherein authenticity of said specified part of the human body is determined based on variations in the characteristics of the electrical output detected by said detection means.

According to another embodiment of the invention, a method is provided which includes arranging an antenna in association with a specified portion of a sensor onto which a specified part of a human body is acted for obtaining data identifying an individual and detecting characteristics of electrical output of an electrical circuit including the antenna when an object other than said specified part of the human body is acted on said specified portion of the sensor, wherein authenticity of said specified part of the human body is determined based on variations in the characteristics of the electric output.

Thus, the present invention provides a variety of methods and apparatus for determining whether an object comprises living tissue. An antenna is provided which is operable to form an electrical circuit with the object. Detection circuitry is provided which is operable to detect at least one parameter corresponding to the electrical circuit. Determination circuitry is provided which is operable to determine whether the object comprises living tissue with reference to the at least one parameter.

A further understanding of the nature and advantages of the present invention may be realized by reference to the remaining portions of the specification and the drawings.

### BRIEF DESCRIPTION OF THE DRAWINGS

Fig. 1 is a schematic view showing an example of a finger authentication device, as one example of a living body determination device of the present invention, built into a fingerprint matching system for individual authentication.
Fig. 2A is a structural cross sectional view of a fingerprint sensor, Fig. 2B shows a sheet antenna, and Fig, 2C shows a loop antenna.
Fig. 3 shows variation in transmission frequency when a person's finger and a false finger are brought close to a fingerprint sensor.
Fig. 4 shows variation in transmission output level when a person's finger and a false finger are brought close to a fingerprint sensor.
Fig. 5 shows variation in transmission phase when a person's finger and a false finger are brought close to a fingerprint sensor.
Fig. 6 shows the state of electromagnetic waves induced from electrical power lines being received by a human body.
Fig. 7 shows a dummy finger placed on a fingerprint sensor.
Fig. 8 shows a dummy finger placed on a fingerprint sensor.

### DETAILED DESCRIPTION OF SPECIFIC EMBODIMENTS

Reference will now be made in detail to specific embodiments of the invention including the best modes contemplated by the inventors for carrying out the invention. Examples of these specific embodiments are illustrated in the accompanying drawings. While the invention is described in conjunction with these specific embodiments, it will be understood that it is not intended to limit the invention to the described embodiments. On the contrary, it is intended to cover alternatives, modifications, and equivalents as may be included within the spirit and scope of the invention as defined by the appended claims. In the following description, numerous specific details are set forth in order to provide a thorough understanding of the present invention. The present invention may be practiced without some or all of these specific details. In addition, well known process operations have not been described in detail in order not to unnecessarily obscure the present invention.

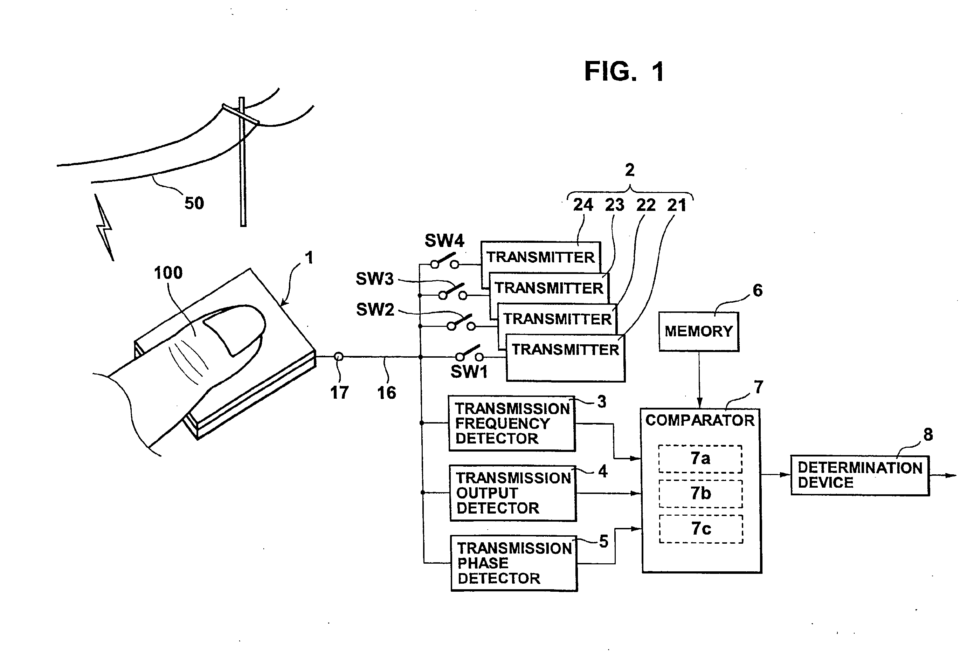

Fig. 1 is a schematic drawing showing an example of a finger authentication device, as one example of a living body determination device of the present invention, built into a fingerprint matching system for individual authentication. Reference numeral 1 is a fingerprint sensor used in a fingerprint authentication system. The fingerprint sensor 1 may be formed from any kind of optical type, pressure sensitive type (e.g., as proposed in Japanese Patent Laid-open No. Hei. 8-68704), electrostatic type, magnetic type, or piezoelectric type, etc. The surface of the fingerprint sensor 1 is provided with a section (specified site) for accommodating a person's finger.

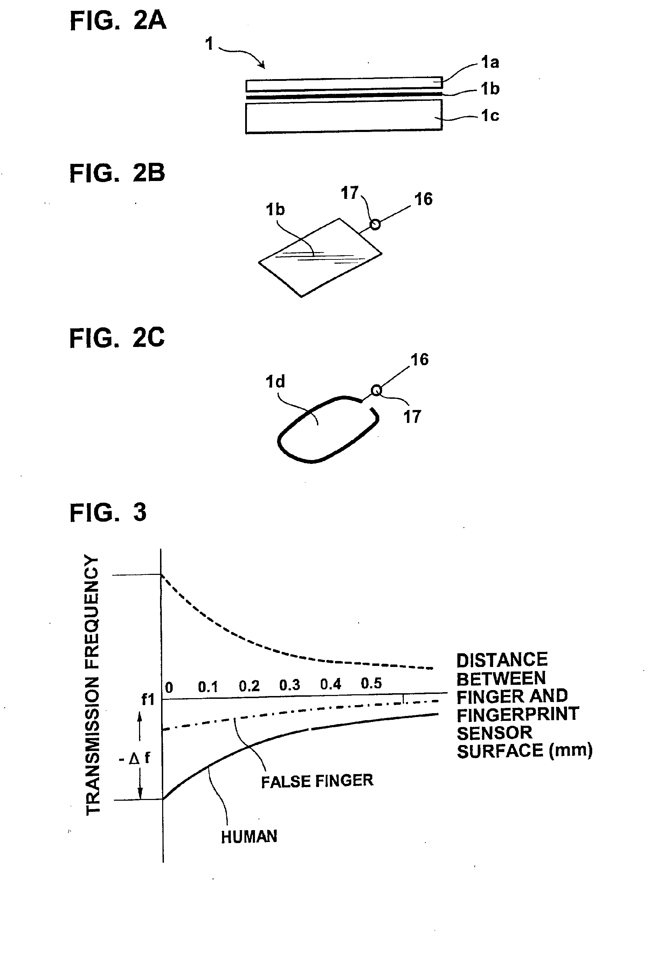

It should be noted that some of the details of fingerprint authentication system of Fig. 1 are merely exemplary and are not to be construed as limiting the invention in any way. In addition, the structure of the fingerprint sensor 1 is not fundamental to the present invention, and so will not described in detail, but as shown in Fig. 2A, a flexible pressure sensitive sheet 1a is provided at an uppermost position, and a sheet antenna 1b is arranged between this pressure sensitive sheet 1a and an electronic circuit substrate 1c on which an electronic circuit including TFTs or transistors has been formed by photolithography on an insulating substrate such as a glass substrate. This sheet antenna 1b can be formed from a spread out conductive film (for example a copper clad laminate) as shown in Fig. 2B separate from the pressure sensitive sheet 1a, but it is also possible to utilize a conductive film deposited on the rear surface of the pressure sensitive sheet 1a constituting the fingerprint sensor 1. The surface area of the sheet antenna 1b can be set to an appropriate size of 0.01 mm² or more, in consideration of the fingerprint sensor 1.

As an alternative to sheet antenna 1b, it is also possible to use a loop antenna 1d as shown in Fig. 2C. This loop antenna 1d can be manufactured using an electrical conductor having a length of at least 0.01 mm. Whichever of sheet antenna 1b and loop antenna 1d are employed, the antenna is connected at electrical connection 17 to an electrical line 16 running to a measurement system that will be described later. According to various embodiments, connection between the antenna and the electrical connection 17 can be achieved using a variety of mechanisms including, for example, a dc connection, an ac connection, an electromagnetic connection, a connection using sound or light, or a wireless connection as appropriate with regard to a variety of factors including, for example, the particular use or place of installation of the system.

Reference numeral 2 indicates a group of transmitters, and in the example shown in Fig. 1 this group comprises four self-oscillation type transmitters 21, 22, 23 and 24 which are designed to oscillate at respectively different fixed reference frequencies f1, f2, f3 and f4. Each self-oscillation type transmitter may be connected to the electrical connection terminal 17 via electrical line 16 and the respective electronic switches SW1, SW2, SW3 and SW4. The electronic switches SW1 - SW4 are turned on and off by a control circuit that is not shown in the drawing. According to a specific embodiment, connection of the four self-oscillation type transmitters 21 - 24 is effected in a random order by this turning on and off of the electronic switches. The self-oscillation type transmitters 21 - 24 may comprise any of a wide variety of oscillators including, for example, a CR oscillator, a Colpitts oscillator, a Hartley oscillator or a phase shift oscillator.

Reference numeral 3 is a transmission frequency detector for detecting the transmission frequency of each transmitter in the transmitter group 2, reference numeral 4 is a transmission output detector for detecting output level (effective value) of each transmitter of the transmitter group 2, and reference numeral 5 is a transmission phase detector for detecting the transmission phase of each transmitter of the transmitter group 2. Reference numeral 6 is a memory for storing first standard data and second standard data described below.

First Standard Data: Frequency difference (frequency variation amount) data and phase difference (phase variation amount) data representing differences in frequency and phase between a signal transmitted by a transmission circuit, in which each transmitter 21, 22, 23 and 24 of the transmitter group 2 includes a sheet antenna 1b, with a person having placed a finger 100 on the fingerprint sensor 1, and a signal transmitted by the same transmitter circuit when a typical false finger has been placed in the fingerprint sensor 1, and signal output level (effective value) data of a signal transmitted by the transmitter circuit when a finger is placed on the fingerprint sensor 1.

Second Standard Data: Data for frequency, output level (effective value) and phase of a signal output from the sheet antenna 1b with a finger 100 of a person that has been affected by electromagnetic waves from power supply lines placed in the fingerprint sensor 1.

Reference numeral 7 is a comparator for, prior to operation of the fingerprint matching system, comparing frequency variation amount, output level (effective level) and phase variation amount obtained based on frequency, output level (effective value) and phase detected using the transmission frequency detector 3, transmission output detector 4 and transmission phase detector 5 with first standard data previously stored in the memory 6, and then comparing with second standard data. Comparator 7 includes a frequency variation amount comparator 7a, an output level comparator 7b and a phase variation amount comparator 7c. Reference numeral 8 is a determination device for determining whether or not the finger 100 placed in the fingerprint sensor 1 is the finger of a (living) person, based on output from the comparator 7.

Reference numeral 50 represents power supply lines such as supply lines or distribution lines that are laid close to people in their living environment, and the significance of this will be described later.

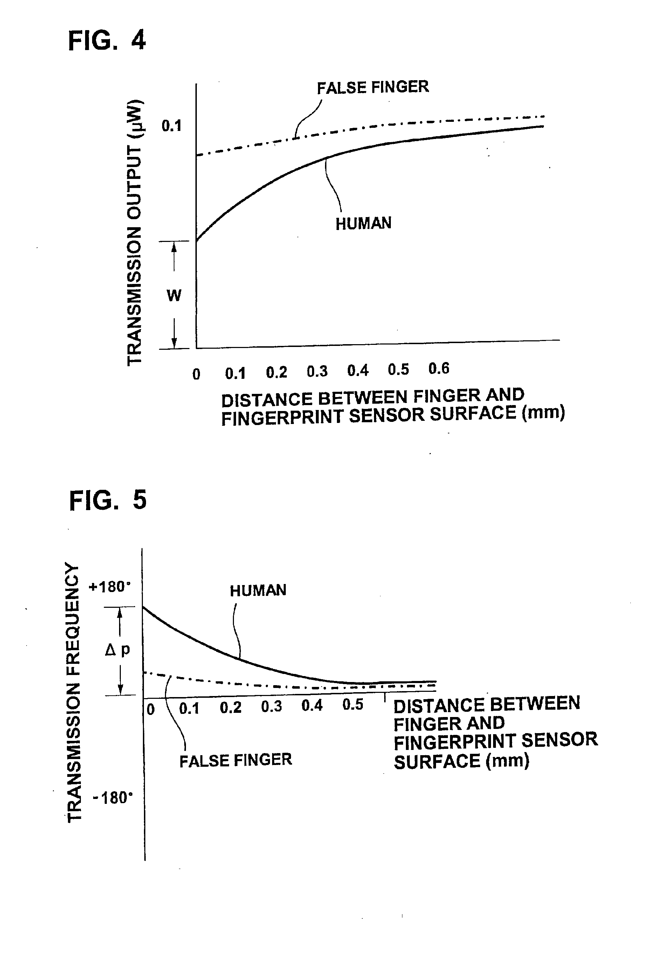

The operation of a specific embodiment of the present invention will now be described. In Fig. 1, if an image of a person's finger 100 is pressed on the surface of the fingerprint sensor 1 is observed in detail with one transmitter (for example self-oscillation type transmitter 21) transmitting, the transmission frequency of the transmitter 21 detected by the frequency detector 3 varies as shown in Fig. 3 according to distance between a fingerprint pattern surface of the finger 100 and the surface (specified site) of the fingerprint sensor 1 being pressed down upon by the finger.

In Fig. 3, the vertical axis represents transmission frequency of the transmitter, while the horizontal axis represents distance between the fingerprint pattern surface of the finger 100 and the surface of the fingerprint sensor 1 being pressed down upon by the finger.

The transmitter 21 self-oscillates at, for example, a reference oscillation frequency f1 = 1 MHz with an output fed back to the input at a phase of at least 180°, and an electrical line 16 is connected to the feedback loop of this transmitter 21. The electrical line 16 is connected to the electrical connection terminal of the sheet antenna 1b as shown in Fig. 2B.

As shown in Fig. 3, as the finger 100 is brought close to the fingerprint sensor 1 the transmission frequency of the transmitter 21 gradually shifts from the reference oscillation frequency f1 (MHz) by an amount of shift (variation amount) Δf from the reference oscillation frequency f1. In an exemplary case where f1 is 1.0 MHz, when the finger 100 is pressed closely against the surface of the fingerprint sensor 1, Δf is -0.2 MHz, and so the transmission frequency becomes 0.8 MHz.

This is because an impedance component (reactance component, inductance component, resistive component) introduced by the body as a dielectric having the finger 100 as its part, is electrically coupled to the circuitry of the transmitter 1 through the sheet antenna 1b, and as a result the transmission frequency, output level (effective value) and phase of the transmitter 21 vary. Fig. 3 shows variation in frequency with a solid line. Depending on the connection point of the transmitter 21, variation in frequency may also move towards the + side, as shown by the dotted line above f1.

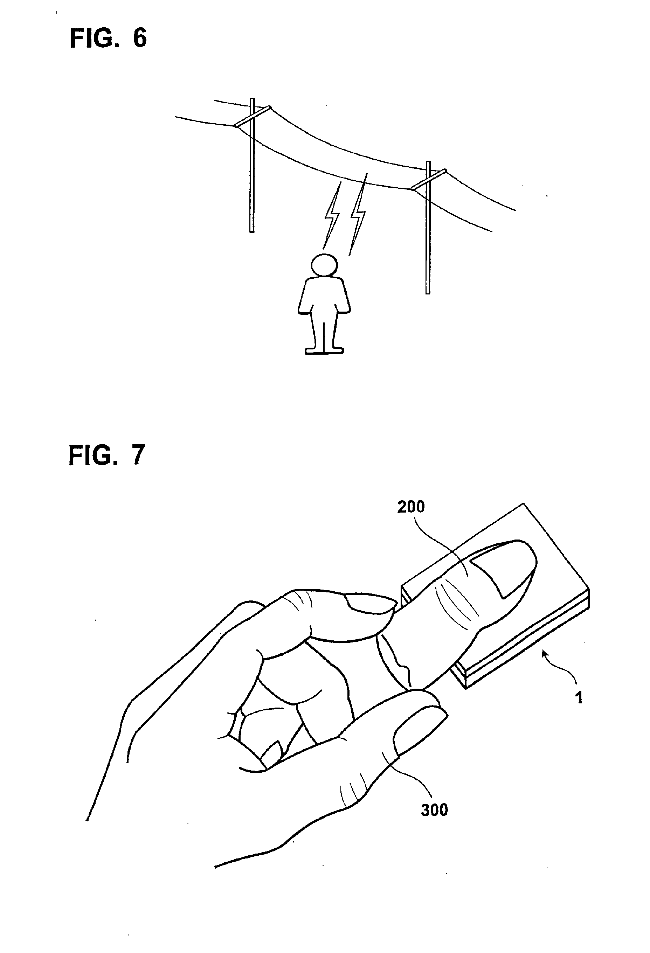

Variations in output level (effective value) and phase from the transmitter due to variation in distance between the finger 100 and a surface (specified site) of the fingerprint sensor 1 being pressed down on by the finger are shown in Fig. 4 and Fig. 5. First standard data stored in the memory 6 is Δf, w and Δp shown in Fig. 3, Fig. 4 and Fig. 5.

This phenomenon can be explained as follows. Observing the side of the fingerprint sensor 1 from the transmitter 21 as an electrical circuit, a human body can be considered equivalent to an enormous dielectric extending from the surface of the fingerprint sensor 1 to the finger 100, wrist, arm and torso, and from a child weighing about 20 kg to an adult weighting in excess of 100 kg, all are considered to be large dielectrics. With this as a basic premise, the present invention accounts for the fact that conductivity of tissue, such as internal organs, skin, blood etc., differs depending on whether a person is living or dead. A difference in this conductivity may be determined with reference to interstitial moisture content.

According to various embodiments, reference oscillation frequencies of the transmitters 21 ∼ 24 are set in advance to different values over an arbitrarily large range. According to one exemplary embodiment, the range is between 10 Hz and 10 GHz, with the reference oscillation frequency of the transmitter 21 being set to 10 Hz, the reference oscillation frequency of the transmitter 22 being set to 1 KHz, the reference oscillation frequency of the transmitter 23 being set to 100 KHz, and the reference oscillation frequency of the transmitter 24 being set to 1 GHz. By manipulating electronic switches SW1 ∼ SW4 during (within or in) a short time, variations in transmission frequency of the respective transmitters 21 ∼ 24 for an object placed at the specified site of the surface of the fingerprint sensor 1 (person's finger or false finger etc.) are detected by the transmission frequency detector 3. If the time taken to switch a transmitter using the electronic switches SW1 ∼ SW4 is, for example, 0.2 seconds, 0.8 seconds will generally be required to switch the four transmitters 21, 22, 23 and 24. Transmission frequency in the case of a false finger varies as shown by the dashed line in Fig. 3.

Transmission frequency, transmission output level (effective level) and transmission phase are respectively detected by the transmission frequency detector 3, transmission output level detector 4 and transmission phase detector 5 for each switched transmitter, and sent to the comparator 7. In the case of a false finger, transmission output (effective value) and transmission phase vary as shown by the dashed line in Fig. 4 and Fig. 5.

In the comparator 7, amounts of variation for frequency and phase, or detected level itself for output level, from the transmission frequency, output level (effective level) and transmission phase respectively detected by the transmission frequency detector 3, transmission output level detector 4 and transmission phase detector 5, are compared with first standard data previously stored in the memory 6 by the frequency comparator 7a, output level comparator 7b and phase variation amount comparator 7c.

It should be noted that in our living environment, high voltage electrical supply lines for a few thousands of volts to a few tens of thousands of volts are found anywhere around the country, and there are power distribution lines in the household environment, but these power supply lines are supplying alternating current which means that electromagnetic waves (50 Hz, 60 Hz) from the power supply lines are supplying routinely induced in the human body. Fig. 6 shows this aspect. Power supply electromagnetic waves emitted from the electrical supply lines are of enormous power, which means that these electromagnetic waves exist in almost all areas and buildings having electric light. Also, noise from these electrical supply lines exists in all countries adopting a power supply system using alternating current, as shown in the table attached to the end of the specification. Since there are hardly any countries performing direct current electrical supply, it is no exaggeration to say that the earth's surface is being covered with electrical supply noise.

This being the case, the human body becomes a large dielectric antenna, and this human body antenna is particularly receptive to low frequencies of 50 Hz and 60 Hz. If the human body is measured directly, power supply frequency noise is inevitably contained. For example, it is a common experience to be able to hear a humming sounds known as a giant boom from a speaker if a person's hands are brought close to the input terminals of an amplifier located in a lecture theatre, but this noise is power supply frequency noise.

Because of this phenomenon, fingerprint matching systems using an AC 100 V power source automatically generate electromagnetic waves from the fingerprint sensor to the periphery. In view of this fact, and according to a specific embodiment of the invention, the electronic switches SW1 ∼ SW4 of Fig. 1 may all be turned off to disconnect the transmitter group 2 from the fingerprint sensor 1. This is because if a person's finger is correctly placed on the fingerprint sensor 1 electromagnetic waves from the power supply lines 50 are received by the human body antenna and output through the sheet antenna 1b of the fingerprint sensor 1. The frequency, effective value and phase of the output signal from this antenna are detected by the transmission frequency detector 3, transmission output level detector 4 and transmission phase detector 5, through the electrical line 16. These values may then be compared to the second standard data (described above) previously stored in the memory 6.

Following comparison of the frequency variation amount, output level (effective value) and phase variation amount acquired by switching the transmitter group 2 described above, with the electronic switches SW1 ∼ SW4 now all in the off state (i.e., with the transmitter group 2 disconnected), frequency, output level (effective level) and phase of a signal output from the sheet antenna 1b of the fingerprint sensor 1 using electromagnetic waves from the power supply line 50 are compared with second standard data previously stored in the memory 6. The determination device 8 synthetically judges comparison results obtained using the transmitter group and comparison results obtained using the power supply lines, and whether a finger being pressed down upon the fingerprint sensor 1 is a person's finger 100 or a false finger is determined by the determination device 8. Various methods can be considered for determination using the determination device 8, and among them there is a simple but strict method in which it is determined that a finger on the fingerprint sensor 1 is a person's finger if differences from reference date obtained for each of electrical characteristics such as frequency, output level and phase fall within respective specified ranges, and that the finger on the fingerprint sensor is a false finger if, for example, a difference for even one of the electrical characteristics does not fall within the specified range. With respect to the method of determination, it is possible to have any determination by varying processing in order to perform determination according to type, use, purpose, etc. of individual authentication system or fingerprint matching system. A method of determination using power supply lines is simple and inexpensive.

An operation for determining authenticity of a finger placed on a fingerprint sensor has been described above, and this operation is executed first of all in individual authentication processing of a fingerprint matching system. This specifics of such an individual authentication processing are not fundamental to the present invention, and so are not described here.

With the above described embodiment, the electronic switches SW1 ∼ SW4 are all switched off when detecting electrical characteristics using electromagnetic waves from the power supply lines, but it is also possible that the self oscillating operation is halted by a control circuit (not shown) without the electronic switches S1 ∼ SW4. Also, embodiments not including such switches or their associated oscillators and relying on the use of externally generated electromagnetic waves are contemplated.

In the above-mentioned embodiment, authenticity of a finger as a living body is determined based on all of the detection results of three electrical characteristics, namely frequency, output level and phase based on all of the detection results for these characteristics, but it is not necessary to use all of these characteristics in determination. It goes without saying that it is also possible to restrict subjects of detection and select characteristics to be used in the determination depending on use, a system, or purpose, etc. That is, any one of these parameters, alone or in combination with any others of the parameters may be used in the determination.

Further, in an environment where it is not possible to use electromagnetic waves from the power supply lines, or an environment where such electromagnetic waves from power supply lines are not used deliberately, it is possible to install at least one external transmitter for oscillation at a fundamental frequency of from 10 Hz to 10 GHz nearby, and to use electromagnetic waves of power propagated through space from this external transmitter and induced in the human body.

Here, a description will be given on applications of the finger determination device as an embodiment of the present invention to determination of a false finger.

Fig. 7 shows an artificial finger 200 as an example of a false finger held by a hand 300 of a person, and being pressed down on the fingerprint sensor 1.

Viewed from the surface of the fingerprint sensor 1, treating the situation as an electrical circuit, the artificial finger 200 is connected as a dielectric between the person (hand 300) and the fingerprint sensor 1. If comparison is made to the case where a person's finger is directly pressed down on the fingerprint sensor 1, transmission characteristics of a transmitter are significantly different. This means that if any kind of substance is inserted between the person's finger and the fingerprint sensor 1, conductivity will noticeably differ, so that it is possible to simply identify the artificial finger 200 by detecting transmission characteristics of the transmitter.

Referring to Fig. 7, if it is assumed that the artificial finger 200 is not an artificial finger but a finger that has been severed from a human body, the conductivity of the finger 200 is different from the conductivity of the finger before it was separated, because the finger has already been separated from the body. Further, a (living) person's hand 300 holding this artificial finger 200 and the severed artificial finger 200 are in partial contact with one another, but viewed as an electrical circuit the two are considered to be separate from each other. This results in that dielectric coupling is caused between the skin of the hand 300 and the skin of the artificial finger 200, and a series connection is formed from the person's hand 300, through a contact site to the artificial finger 200. This condition is clearly very different from the case where a living person brings his finger into contact with the fingerprint sensor 1, and exhibits with the result that significantly different transmitter characteristics will appear. The reason why this difference appears so large is that at places not separated significantly from a measurement point (surface of the fingerprint sensor 1), that is, at places about 4 to 10 cm away, discontinuous coupling to the electrical circuit arises, and since particularly large differences arise, these differences can be used in discovering a false finger which is extremely beneficial.

For further consideration, a case will be assumed not where a finger has been severed from a human body, but where an arm has been severed from a human body but a finger at the end of the arm is pressed down on the fingerprint sensor 1. Since the hands of a person (living person) carrying the severed arm and the finger at the end of the severed arm are at least 30 cm part coupling of two bodies at a distance separated to that extent is not considered to be an electrical connection. The reason for this is simple . If it is assumed that capacitance of a finger itself is 10 µF, a 10 µF series connection is formed resulting in that capacitance including that connection as viewed from the fingerprint sensor 1 will become 5 µF. If it is assumed that capacitance to an arm is 100 µF, there will only be a connection of a resultant capacitance of at most 50 µF with 100 µF connected.

That is, at a surface reference of the fingerprint sensor, if a human body of a person and a false finger are connected at a position close to the fingerprint sensor, additional coupling capacitance will exist at a small distance so that a large difference will appear from the case where a person's finger is placed. On the other hand, if the human body of the person and the false finger are coupled at a place remote from the fingerprint sensor, the capacitance of an artificial body becomes large. This means that only a large capacitance coupling is considered to be coupling, or unless an object having a larger capacitance than the person is needed. For coupling with a large surface area binding two arms with a string is needed. Accordingly, this is extremely beneficial as false fingerprint countermeasures. In any event, the difference between a living person's finger and a false finger is evident.

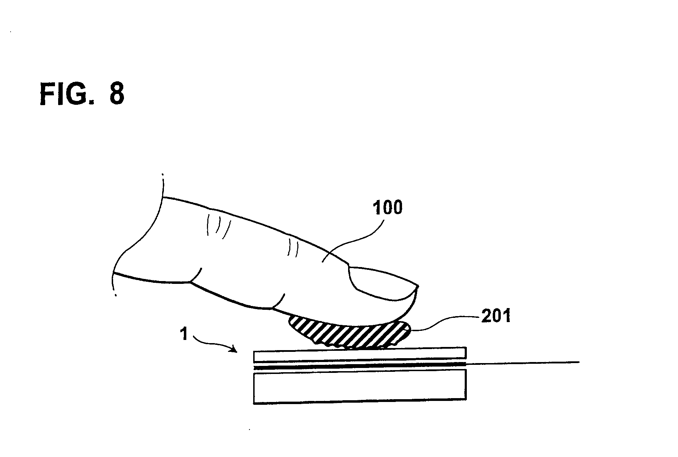

Fig. 8 shows another application of the finger determination device of the present invention to determination of a false finger. Fig. 8 shows an artificial finger 201 with another person's fingerprint pattern formed on the tip of a person's finger 100 which is pressed down on the fingerprint sensor 1.

In this case, contact capacitance between the finger 100 and the artificial finger 201 constitutes a problem, and there appears a large difference from the case where only the finger 100 is passed down on the fingerprint sensor 1. With experimentation, difference in transmission characteristics is plainly evident, even if a single sheet of paper (having a thickness of about 10 microns) is interposed between the finger 100 and the fingerprint sensor 1. Therefore an artificial finger will be uncovered even if a finger cover with a false fingerprint is formed of any sheet material having a thickness of 10 microns. This is similar to technology capable of measuring cracks, even if a ceramic product once cracked is bonded using any kind of adhesive. Even if it appears that the ceramic product has been unified on its outer appearance, it is possible to electrically detect faults. In short, in determining authenticity of a finger, it is enough to detect whether a finger in question has fixed conductivity as being continuous with a human body. By using this determination method, it is possible to confirm a human body at extremely minimum cost in a purely electronic manner, which enables construction of a required system, and building into a machine.

A description has been given on an example of application of a living body determination device of the present invention to a fingerprint matching system, but the present invention is applicable not only to fingers as a specified part of a human body, but also to other specific part of the human body such as wrist vascularity patterns, retina, or iris etc which are used as biometrics. In case of individual authentication using the wrist vascularity, an antenna may be arranged at a position where a wrist is pressed down in an overall wrist vascularity measurement device. In individual authentication using the retina or iris, an antenna may be arranged on a fixed platform fixing a position of the eye in an iris observation device for observing the retina or iris of an eye of a person.

**Table 1**

| Country | Voltage (AC) V | Frequency (Hz) |
|---|---|---|
| Japan | 100 | 50/60 |
| China | 110/220 | 50 |
| Korea | 110/220 | 60 |
| Hong Kong | 200/220 | 50 |
| Taiwan | 110 | 60 |
| Thailand | 220/240 | 50 |
| Philippines | 110/115/220 | 60 |
| Indonesia | 127/220 | 50 |
| Singapore | 110/230 | 50 |
| India | 220/230/250 | 50 |
| Saudi Arabia | 127/220/230 | 50/60 |
| Australia | 240 | 50 |
| New Zealand | 230/240 | 50 |
| America | 10/117/120 | 60 |
| Canada | 120/240 | 60 |
| Mexico | 125 | 60 |
| Brazil | 127/220 | 60 |
| Argentina | 220 | 50 |
| Chile | 220 | 50 |
| Great Britain | 240 | 50 |
| France | 127/220 | 50 |
| Germany | 127/220 | 50 |
| Italy | 110/220 | 50 |
| Spain | 110/220 | 50 |
| Greece | 220 | 50 |
| Austria | 220 | 50 |
| Sweden | 110/220 | 50 |
| Russia | 127/220 | 50 |
| Kenya | 240 | 50 |

The present invention detects variations in electrical characteristics caused by variation in conductivity due to presence or absence of a human body as a subject to be judged, at the time of judging authenticity of a specified part of the human body in order to judge authenticity of a specified part of the human body with an extremely simple operation and means, and also at low cost. Accordingly, if the human body determination device of the present invention is applied to determination of authenticity of a finger, as a part of the human body, it is possible to solve the problem of artificial fingerprints in a fingerprint matching system using a fingerprint sensor, and it is possible to reliably prevent acts of illegal access or acts of impersonation in the field of individual authentication using a fingerprint sensor. In case of applying the present invention to a fingerprint matching system using a fingerprint sensor, the present invention is not limited to any particular type of fingerprint sensor. That is, optical type, pressure sensitive type, electrostatic type, magnetic type or piezoelectric type sensors are all within the scope of the invention.

While the invention has been particularly shown and described with reference to specific embodiments thereof, it will be understood by those skilled in the art that changes in the form and details of the disclosed embodiments may be made without departing from the spirit or scope of the invention. In addition, although various advantages, aspects, and objects of the present invention have been discussed herein with reference to various embodiments, it will be understood that the scope of the invention should not be limited by reference to such advantages, aspects, and objects. Rather, the scope of the invention should be determined with reference to the appended claims.

## Claims

1. A living tissue determination device, comprising:
an antenna arranged in proximity to a sensor;
first circuitry for detecting at least one characteristic of an electrical circuit including the antenna and an object proximate the sensor; and
second circuitry for determining whether the object comprises living tissue with reference to the at least one characteristic.

2. The device of claim 1 wherein the sensor is a fingerprint sensor used for individual authentication, the sensor having a surface upon which a finger may be pressed.

3. The device of claim 2 wherein the antenna has a spread out conducting surface whose total surface area is at least 0.01 mm².

4. The device of claim 2 wherein the antenna comprises an electrical wire having a length of at least 0.01 mm.

5. The device of any one of claims 1 to 4 wherein the antenna is connected to an external electrical line via any of a direct current connection, an alternating current connection, an electromagnetic connection, a connection using light, a sound connection, or a wireless connection.

6. The device of any one of claims 1 to 5 wherein the antenna is connected to an external electrical line through an electrical connection terminal, and the electrical connection terminal is connected with a human body through direct current connection, alternating current connection, electromagnetic reflection connection, light reflection comiection or sound reflection connection.

7. The device of any one of claims 1 to 6 wherein the first circuitry comprises a plurality of self-oscillating type transmitters, determining whether the object comprises living tissue being done by the second circuitry with reference to variation in any of transmission frequency, transmission output level and transmission phase of the self-oscillating type transmitters.

8. The device of claim 7 wherein the first circuitry is operable to employ the plurality of self-oscillating type transmitters sequentially.

9. The device of claim 1 or claim 2 wherein the antenna is operable to receive an externally generated electromagnetic signal, and the first circuitry is operable to detect any of a frequency, output level or phase of a resulting signal induced in a human body by the externally generated electromagnetic signal.

10. The device of clam 1 or claim 2 wherein the first circuitry is operable to detect a frequency or noise level of a signal induced in a human body by propagation of electrical power through space from electrical power lines.

11. The device of claim 7 or claim 8 wherein the second circuitry is operable to use any of an amount of variation in frequency, a transmission output level or a transmission phase of an output of a self-oscillating type transmitter for determining whether the object comprises living tissue.

12. The device of claim 1 wherein the sensor is a vascularity measuring device used in individual authentication upon which a wrist is pressed.

13. The device of claim 1 wherein the sensor is a fixed platform for fixing a position of an eye in an iris observation device used in individual authentication.

14. An individual authentication device incorporating the device of any one of claims 1 to 14.

15. A method for determining whether an object in proximity to a sensor comprises living tissue, the method comprising:
arranged arranging an antenna in proximity to a sensor;
detecting at least one characteristic of an electrical circuit including the antenna and the object; and
determining whether the object comprises living tissue with reference to the at least one characteristic.

16. A device for determining whether an object comprises living tissue, comprising:
an antenna operable to form an electrical circuit with the object;
detection circuitry operable to detect at least one parameter corresponding to the electrical circuit; and
determination circuitry operable to determine whether the object comprises living tissue with reference to the at least one parameter.

17. The device of claim 16 wherein the antenna comprises either of a sheet antenna and a loop antenna.

18. The device of claim 16 wherein the detection circuitry comprises at least one of a frequency detector, an output level detector, and a phase detector, and the at least one parameter comprises frequency, voltage level, and phase.

19. The device of claim 16 further comprising at least one transmitter operable to generate a signal, the detection circuitry being operable to detect the signal via the electrical circuit.

20. The device of claim 19 wherein the at least one transmitter comprises a plurality of transmitters each being **characterized by** a different frequency, the device further comprising switching circuitry for alternating connecting the transmitters to the antenna.

21. The device of claim 16 wherein the antenna is coupled to the detection circuitry via one of a direct current connection, an alternating current connection, an electromagnetic connection, a connection using light, a sound connection, or a wireless connection.

22. The device of claim 16 wherein the detection circuitry is operable to detect electromagnetic radiation via the electrical circuit, the electromagnetic radiation being generated externally to the device.

23. The device of claim 16 wherein the determination circuitry comprises comparison circuitry and memory, the comparison circuitry being operable to compare the at least one parameter to at least one previously stored value from the memory.

24. The device of claim 23 wherein the determination circuitry further comprises a processing circuitry for determining whether the object comprises living tissue with reference to the comparison between the at least one parameter and the at least one previously stored value.

25. The device of claim 24 wherein the processing circuitry is operable to indicate that the object is not living tissue when the at least one parameter differs from the at least one previously stored value by more than a predetermined amount.

26. A biometric authentication system comprising the device of claim 16.

27. The biometric authentication system of claim 26 wherein the system comprises one of a fingerprint recognition system, a wrist vascularity measurement system, a retinal scanner, and an iris scanner.
